# EUROPÄISCHE PATENTSCHRIFT

(11) **EP 2 321 300 B1**
(45) Veröffentlichungstag und Bekanntmachung des Hinweises auf die Patenterteilung: **07.12.2011**
(21) Anmeldenummer: 09775561.5
(22) Anmeldetag: 14.07.2009
(51) Int. Cl.: C07D 401/14, A61K 31/409

(54) **CHLOROPHYLLKATABOLITEN**
CHLOROPHYLL CATABOLITES
CATABOLITES DE CHLOROPHYLLE

(30) Priorität: 22.07.2008 AT 11332008
(43) Veröffentlichungstag der Anmeldung: 18.05.2011
(73) Patentinhaber: Universität Innsbruck, 6020 Innsbruck (AT)
(72) Erfinder: KRAEUTLER, Bernhard, A-6020 Innsbruck (AT); MOSER, Simone, A-6176 Völs (AT)
(74) Vertreter: Vögele, Andreas
(86) Internationale Anmeldenummer: PCT/AT2009/000274
(87) Internationale Veröffentlichungsnummer: WO 2010/009487

(56) Entgegenhaltungen:
- WO-A-2007/030848
- LEHNINGER, A.L.: "Prinzipien der Biochemie" 1994, SPEKTRUM AKADEMISCHE VERLAG GMBH , BERLIN , XP002544558 Struktur des Bilirubins Seite 814
- MOSER S ET AL: "A yellow chlorophyll catabolite is a pigment of the fall colors" PHOTOCHEMICAL AND PHOTOBIOLOGICAL SCIENCES, ROYAL SOCIETY OF CHEMISTRY, CAMBRIDGE, GB, Bd. 7, Nr. 12, 17. Oktober 2008 (2008-10-17), Seiten 1577-1581, XP009122363 ISSN: 1474-905X

## Beschreibung

Die Erfindung betrifft Chlorophyllkataboliten, die Verwendung und Herstellung dieser Chlorophyllkataboliten sowie die Gewinnung dieser Chlorophyllkataboliten aus pflanzlichem Material.

In der WO 2007/030848 A2 wird die Gewinnung und Charakterisierung sogenannter nichtfluoreszierender Chlorophyllkataboliten (NCC) beschrieben. Die so gewonnenen NCC zeigen eine außergewöhnliche antioxidative Wirkung einerseits und eine medizinische Wirkung, insbesondere in der Therapie und Prävention von bestimmten Krebserkrankungen, andererseits. Die in der WO 2007/030848 A2 beschriebenen NCC können aus pflanzlichem Material, insbesondere senesziertem oder gereiftem Pflanzenmaterial gewonnen werden.

Überraschenderweise konnte nunmehr gefunden werden, dass eine neuartige Verbindung 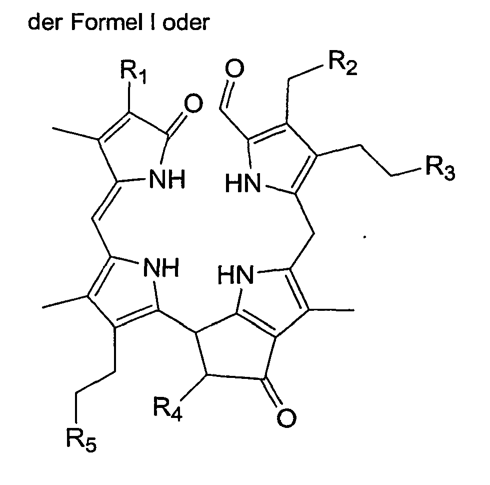 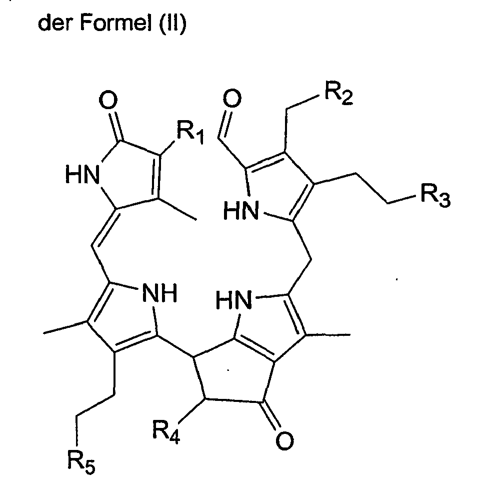 oder Stoffgemische daraus, oder ein pharmakologisch, dermatologisch oder kosmetisch verträgliches Salz, Solvat oder Derivat davon, wobei R₁ bis R₅ ausgewählt ist aus den Resten R₁: -Alkyl, -Vinyl, -CHOH-CH₂OH, -CH(OAcyl)-CH₂OH, -CHOH-CH₂(OAcyl),
-CH(OAcyl)-CH₂(OAcyl), 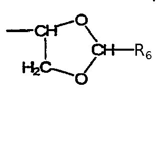 mit R₆ = -Alkyl oder -Aryl,
R₂: -H, -OH, -OAlkyl, -OAcyl, Saccharidreste, acylierte Saccharidreste
R₃: -H, -OH, -OAlkyl, -OAcyl,-OAcyl acyliert, Saccharidreste, acylierte Saccharidreste
R₄, R₅: -COOH, -Carbonsäureester
deutlich verbesserte Eigenschaften gegenüber NCC hinsichtlich ihrer antioxidativen Wirkung aufweisen.

Dabei überrascht insbesondere, dass die neuartigen Verbindungen der Formel 1 oder II durch Oxidation von NCC hergestellt werden können und dabei selbst zu deutlich besseren Antioxidantien als die NCC werden. Erste Untersuchungen haben außerdem gezeigt, dass Verbindungen der Formel I oder der Formel II wirksame Arzneimittel darstellen. Insbesondere hat es sich gezeigt, dass die neuartigen Verbindungen der Formel I oder Formel II wirksam sind bei der Bekämpfung bzw. Prophylaxe von Krebserkrankungen. Die Wirksamkeit hat sich insbesondere bei Krebserkrankung aus der Gruppe Hautkrebs, Brustkrebs, Lungenkrebs, Darmkrebs oder Leukämie erwiesen.

Erste Untersuchungen der chemischen Eigenschaften an Verbindungen der Formel I oder II zeigen, dass diese antioxidative und konservierende Eigenschaften etc. aufweisen. Verbindungen der Formel I und II zeigen keine toxischen Wirkungen, was auch in ihrem natürlichen Vorkommen in der Nahrungskette (in Pflanzenbestandteilen) gestützt wird. Daher wird die Verwendung der Verbindungen der Formel I oder II als Antioxidationsmittel, Konservierungsmittel, Nahrungsergänzungsmittel oder kosmetische Mittel vorgeschlagen.

Bisher konnten zwei Verfahren zur Gewinnung der Verbindungen der Formel I oder II gefunden werden.

Das erste Verfahren zur synthetischen Herstellung von Verbindungen der Formel I oder der Formel II oder Stoffgemischen daraus umfasst die Oxidation einer Verbindung der Formel III 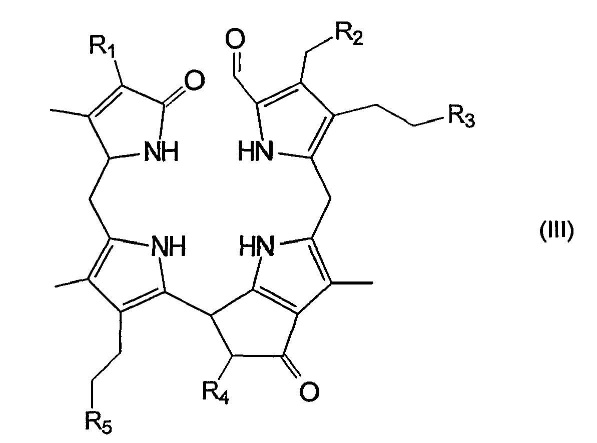 wobei R₁, R₂, R₃, R₄ und R₅ dieselben Reste sind wie die Reste R₁, R₂, R₃, R₄ und R₅ in der Verbindung der Formel (I) oder (II).

Verbindungen der Formel III als Edukte können beispielsweise nach einem in der WO 2007/030848 A2 beschrieben Verfahren hergestellt werden. Die Oxidation der Verbindung der Formel III zu Verbindungen der Formel I oder II kann durch ein geeignetes Oxidationsmittel durchgeführt werden. Geeignete Oxidationsmittel umfassen beispielsweise Verbindungen aus der Gruppe der Benzochinone. Ein geeignetes Benzochinon ist beispielsweise 2,3-Dichlor-5,6-dicyano-p-benzochinon (DDQ). Es sind aber auch andere Oxidationsmittel denkbar, die dem Fachmann geläufig sind.

Um eine hohe Ausbeute zu erzielen kann in einer ersten Variante die Oxidation der Verbindung der Formel III günstigerweise in Lösung bei verhältnismäßig niedrigen Temperaturen erfolgen. Geeignete Temperaturen liegen bei unterhalb von 40 °C, vorzugsweise unterhalb von 0 °C, besonders bevorzugt unterhalb von -30°C. Geeignete Lösungsmittel sind idealerweise solche, die inert sind sowohl gegenüber dem eingesetzten Oxidationsmittel als auch gegenüber dem Edukt (Verbindung der Formel III) und den Produkten (Verbindungen I und II). Aufgrund der amphiphilen Eigenschaften von Verbindungen der Formel I oder der Formel II eignen sich polare, apolare, organische oder anorganische Lösungsmittel.

In einer zweiten Variante lassen sich insbesondere für einen kleineren Maßstab Verbindungen der Formel III auch durch Oxidation mit gasförmigen Oxidationsmittel, insbesondere Sauerstoff wie Luftsauerstoff, in Verbindungen der Formel I oder II überführen. Hierfür ist es günstig wenn die Verbindung der Formel III auf ein Trägermaterial aufgebracht, beispielsweise adsorbiert wird. Durch das Aufbringen auf das Trägermaterial wird die Verbindung der Formel III dem gasförmigen Oxidationsmittel besser zugänglich gemacht, da die dem Oxiationsmittel zugängliche Oberfläche vergrößert wird. Die Reaktionsgeschwindigkeit kann deutlich erhöht werden, wenn die Verbindung der Formel III elektromagnetischer Strahlung, wie Licht, insbesondere im nahem Ultraviolett (UV), d.h. im Wellenlängenbereich von etwa 300 nm bis 400 nm und gegebenenfalls im sichtbaren Wellenlängenbereich (d.h. im Bereich von etwa 400 bis 800 nm) ausgesetzt wird.

Neben dieser präparativen Methode zur Gewinnung von Verbindungen der Formel I oder II besteht auch die Möglichkeit, derartige Verbindungen in einem zweiten Verfahren aus natürlichem, pflanzlichem Material zu gewinnen. Insbesondere aus Pflanzenblättern oder Pflanzenbestandteilen mit grüner oder vormals grüner Färbung kann man durch Extraktion des pflanzlichen Materials mit einem Lösungsmittel zu einem Rohextrakt, anschließender Chromotographie dieses Extraktes und anschließendem Abziehen des Lösungsmittels und gegebenenfalls einer Kristallisation des erhaltenen Rückstandes Verbindungen der Formel I oder II oder Gemische daraus gewinnen.

Bei den Verbindungen bei denen R₁ = 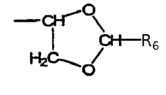 (mit R₆ = -Alkyl oder -Aryl), -CH(OAcyl)-CH₂OH, -CHOH-CH₂(OAcyl)
oder -CH(OAcyl)-CH₂(OAcyl) ist, ging man ursprünglich von einer Verbindung aus bei der R₁ gleich -CHOH-CH₂OH ist. Anschließend erfolgte die Bildung des zyklischen Acetals durch Umsetzen mit dem Aldehyd R₅-CHO bzw. des Mono- oder Diesters durch (einfache oder zweifache) Veresterung des Diols.

Weitere Details der Erfindung, vorteilhafte Ausgestaltungen sowie günstige Alternativen werden im Folgenden unter anderem unter Zuhilfenahme der Figuren erläutert.

Es zeigen
- Fig. 1: schematisch die wichtigsten Reaktionsschritte des Chlorophyllabbaus von Chlorophyll a und Chlorophyll b bis hin zu NCC in seneszenten Blättern,
- Fig. 2: HPLC Chromatogramme (A, B) eines Extraktes seneszenter Blätter, sowie UV/VIS Spektren (C, D) der hervorgehobenen HPLC Fraktionen von A und B,
- Fig. 3a-3c: UV/VIS Spektren zweier Vertreter von Verbindungen der Formel I und der Formel II, deren Strukturformeln in Figuren 3b und 3c dargestellt sind,
- Fig. 4: schematisch den Oxidationsschritt eines NCC zu einer Verbindung der Formel I,
- Fig. 5a: eine Formel eines NCC,
- Fig. 5b: die Nummerierung der einzelnen Atome eines NCC (abgeleitet von der Nummerierung von Chlorophyll gemäß IUPAC),

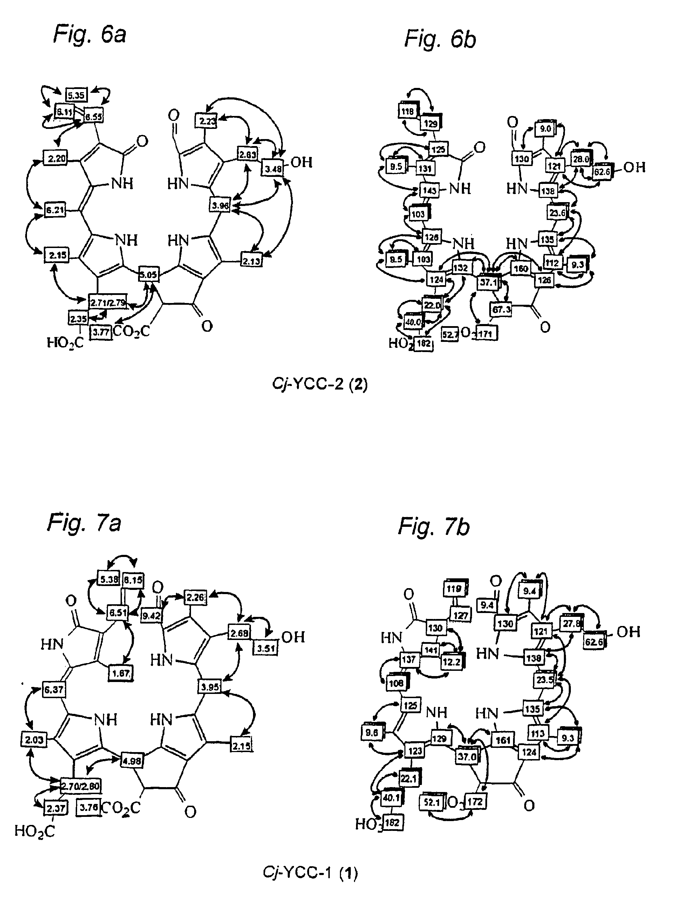

- Fig. 6a: ¹H-Signale eines 500 MHz ¹H-NMR Spektrums einer Verbindung der Formel I,
- Fig. 6b: ¹³C-Signale aus ¹H,¹³C-heteronuklearer Korrelation (in HSQC und HMBC Spektren) einer Verbindung der Formel I,
- Fig. 7a, 7b: die Spektren gemäß Fig. 6a und 6b für eine Verbindung der Formel II und
- Fig. 8: ein Diagramm zur Demonstration der antioxidativen Wirkung eines Beispiels einer Verbindung der Formel I im Vergleich zu Bilirubin.

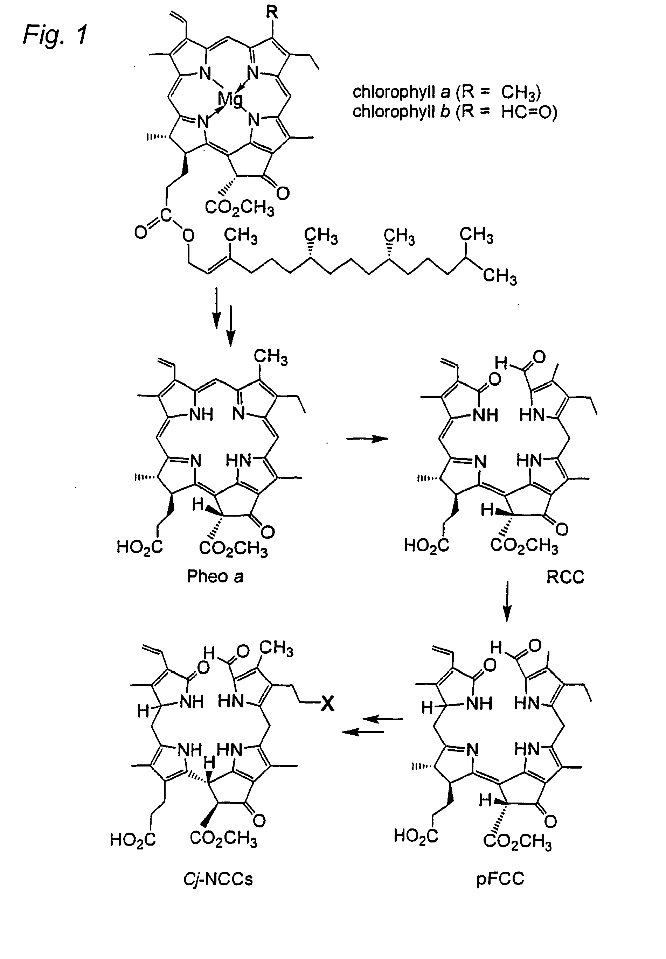

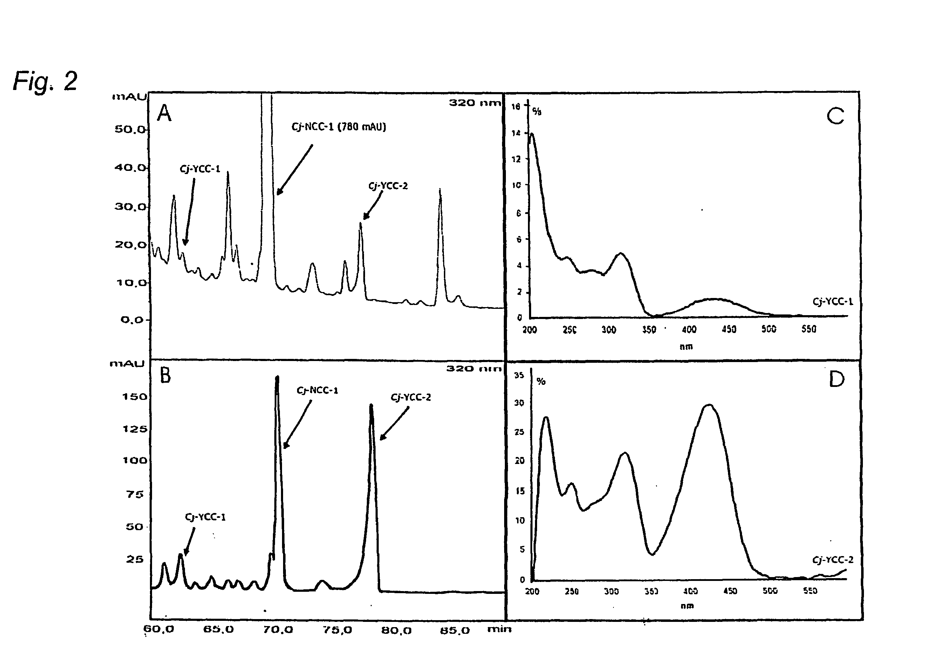

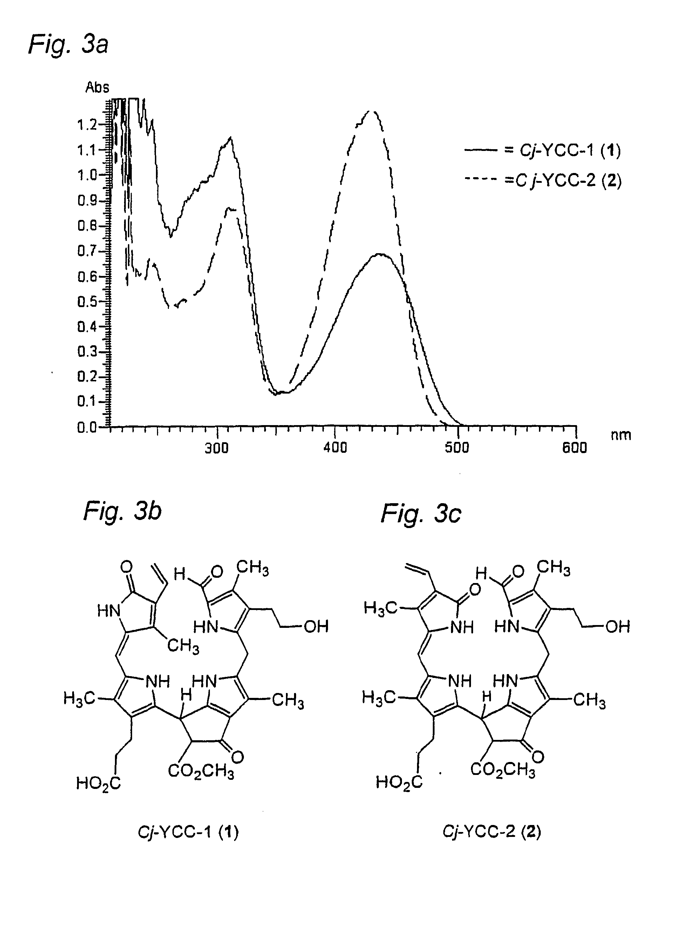

In der Fig. 1 sind schematisch einzelne Reaktionsschritte des Chlorophyllabbaus von Chlorophyll a bzw. Chlorophyll b in *Cercidiphyllum japonicum* beschrieben. Der Abbau erfolgt in mehreren Reaktionsschritten, beginnend zunächst bei Chlorophyll a bzw. Chlorophyll b. Nach mehreren Abbauschritten gelangt man zunächst zum Zwischenprodukt Pheophorbid a (Pheo a). Mittels einer Ringöffnung wird Pheophorbid a in einen roten Chlorophyllkataboliten RCC abgebaut, der wiederum in weiterer Folge zu einem primären fluoreszierenden Chlorophyllkataboliten pFCC und schließlich in sogenannte nichtfluoreszierende Chlorophyllkataboliten NCC umgewandelt wird. In Anlehnung an den Ursprung des entsprechenden NCC von *Cercidiphyllum japonicum* werden diese NCC als Cj-NCC bezeichnet. Wenn der in Fig. 1 mit X bezeichnete Rest ein OH-Rest ist, spricht man von Cj-NCC-1 und wenn der mit X bezeichnete Rest H ist von Cj-NCC-2. NCC bilden einen hervorragenden Ausgangsstoff zur synthetischen Gewinnung von Verbindungen der Formel I oder II. NCC wurden im vorliegenden Fall aus gelben Blättern von *Cercidiphyllum japonicum* gewonnen. Hierfür wurden 500g Frischgewicht an Blättern von *Cercidiphyllum japonicum* gesammelt und zur Lagerung mit flüssigem Stickstoff versetzt. Die Gewinnung von Cj-NCC 1 und *Cj*-NCC-2 erfolgt analog dem in der WO 2007/030848 A2 beschriebenen Verfahren, weshalb hierauf verwiesen wird und die Gewinnung nur sehr kurz erläutert wird. Zunächst wurden Extrakte aus den gefrorenen Blättern gebildet, die anschließend mittels HPLC (high performance liquid chromatography) analysiert wurden. Dabei konnten zwei gelbe Verbindungen isoliert werden, wie aus den Diagrammen der Fig. 2 ersichtlich ist. Es handelt sich dabei um die als *Cj*-YCC-1 und *Cj*-YCC-2 (siehe Fig. 3b bzw. Fig. 3c) bezeichneten Verbindungen, die Beispiele der Verbindungen der Formel II bzw. I darstellen. Die Diagramme A und B der Fig. 2 zeigen dabei HPLC Chromatogramme mit Detektionswellenlänge 320 Nanometer. Aufgetragen ist eine Signalintensität (mAU) gegen die Retentionszeit in Minuten. Die Diagramme C und D zeigen UV/Vis Spektren, der in den Diagrammen A und B mit *Cj*-YCC-1 bzw. *Cj*-YCC-2 beschriebenen Fraktionen. Im Vergleich zu den vorhandenen *Cj*-NCC ist die Menge an *Cj*-YCC in den Blättern verhältnismäßig gering.

Parallel zu diesem analytischen Experiment wurde *Cj*-YCC-2 aus dem Extrakt der Blätter isoliert, welches mittels Chromatographie auf einer Silikagelsäule aufgearbeitet wurde. Fraktionen von *Cj*-NCC-1 wurden gesammelt und durch Fällung aus Dichlormethanlösung durch Zugabe von Hexan ausgefällt. Daraus ergab sich eine Ausbeute von 479 mg *Cj*-NCC-1. In ähnlicher Weise wurde der rohe orangefarbene feste Rückstand von *Cj*-NCC-2 und *Cj*-YCC-2 (zusammen 56,9 mg) mittels MPLC (Medium Pressure Liquid Chromatography) weiter gereinigt, um reine Proben an *Cj-*YCC-2 (27 mg) und *Cj*-NCC-1 (25 mg) zu gewinnen, welche spektroskopisch identifiziert wurden.

In einer präparativen Studie wurden 30 mg *Cj*-NCC-1 auf 3 g Silikagel 60 aus einer Lösung in 50 ml Dichlormethan absorbiert. Anschließend wurde das Lösungsmittel durch Filtration entfernt. Nach Trocknung wurde das getrocknete und leicht gelbliche Pulver elektromagnetischer Strahlung ausgesetzt, und zwar dem Licht einer 100 Watt Wolfram-Glühbirne, während das Silikagel mit adsorbiertem *Cj*-NCC-1 ständig in Anwesenheit von Luft gerührt wurde. Nach 10 Stunden und 15 Minuten wies das Pulver eine orange Färbung auf. Anschließend erfolgte Extraktion des orangefarbenen Pulvers mittels 25 ml Methanol. Die Lösung wurde unter reduziertem Druck mittels eines Rotationsevaporators auf 1,5 ml aufkonzentriert. Mittels HPLC konnte einerseits *Cj*-NCC-1 zurückgewonnen werden, andererseits zeigte sich ein gelbes Hauptreaktionsprodukt, welches mittels Lyophilisierung als gelbes Pulver in einer Ausbeute von 16,0 mg isoliert werden konnte. Die Charakterisierung erfolgte spektroskopisch und zeigte als Reaktionsprodukt *Cj*-YCC-2.

In einem Herstellungsverfahren in größerem Maßstab wurde *Cj*-NCC-1 (193 mg bzw. 300 µMol) in 150 ml Dichlormethan gelöst und in eine Suspension von 19,3 g Silikagel 60 in 200 ml Dichlormethan eingetragen und auf dem Silikagel adsorbiert. Das Lösungsmittel wurde mittels Filtration entfernt und der trockene, leicht gelbe Rückstand in Pulverform einer 100 Watt Wolfram Lampe unter gleichzeitigem Rühren Luftsauerstoff ausgesetzt, sodass eine Oxidation erfolgte. Nachdem das Pulver eine orange-rote Färbung angenommen hatte, wurde eine geringe Menge entnommen und mittels HPLC analysiert. Die Analyse ergab eine Reihe unterschiedlicher UV/VIS aktiver Komponenten, darunter eine gelbe Fraktion von *Cj*-YCC-1 (Retentionszeit 15 Minuten) eine farblose Fraktion *Cj*-NCC-1 (Retentionszeit 17 Minuten) und eine gelbe Fraktion *Cj*-YCC-2 (Retentionszeit 22 Minuten). Das Reaktionsgemisch wurde mittels MPLC(Mitteldruck-Säulenchromatographie) aufgetrennt und nach Aufarbeitung wurden 33,5 mg *Cj*-NCC-1 (52 µMol bzw. Ausbeute 17,4 %) erhalten, und zwei gelbe Pigmente, die als reine Pulver isoliert wurden. Die zwei gelben Pigmente unterteilen sich einerseits in 29,2 mg (45 µMol oder Ausbeute 15,1 %) *Cj*-YCC-1 und 30,3 mg (47 µMol oder 15,7 % Ausbeute) *Cj*-YCC-2 die spektroskopisch identifiziert wurden.

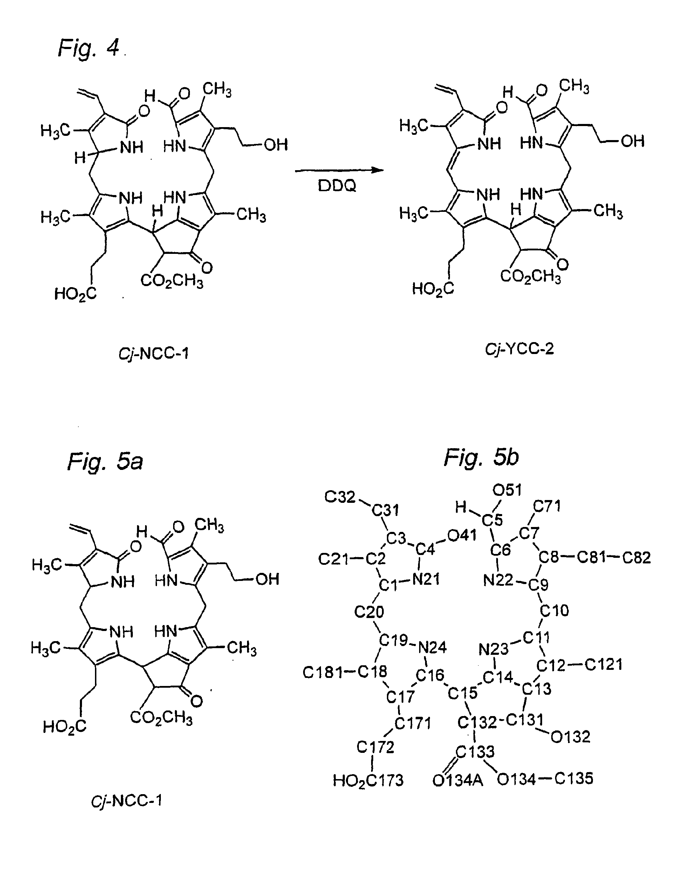

In einem alternativen Verfahren konnte das im Vergleich zu *Cj*-YCC-1 geringfügig weniger polare *Cj*-YCC-2 in einer Ausbeute von 48 % selektiv gewonnen werden, mittels Oxidation von *Cj*-NCC-1 mit einem Benzochinon und zwar mit 2,3-Dichlor-5,6-dicyano-p-benzochinon (DDQ). Der Verfahrensablauf ist in Fig. 4 schematisch dargestellt. Bei - 70° C wurden zu einer Lösung von 50 mg *Cj*-NCC-1 in 5 ml sauerstofffreiem Aceton und 44,4 µl Essigsäure 19,5 mg DDQ in 2,5 ml gekühltem Aceton mittels einer Spritze langsam zugefügt. Die resultierende rote Reaktionslösung wurde über einen Zeitraum von zwei Stunden auf - 47° Celsius gebracht. Die kalte Reaktionslösung wurde mit 192 mg Natriumacetat versetzt und bei einer Temperatur von - 50° Celsius für 30 Minuten gehalten. Die Reaktionslösung wurde anschließend mit 250 ml 0,1 Mol/l Phosphatpuffer mit pH 7 neutralisiert und an einer Sep-Pak Kartusche entsalzt. Die rohe Reaktionsmischung wurde als Festkörper erhalten und in weiterer Folge in Methanol gelöst. Anschließend erfolgte Zugabe und Reaktion mit Trifluoressigsäure für 4 Minuten bei Raumtemperatur. Die Mischung wurde anschließend mit einem Phosphatpuffer pH 7 neutralisiert. Nach Aufarbeitung konnten 52,2 mg feste Roh-Reaktionsmischung erhalten werden. Dieses Rohprodukt wurde mittels MPLC auf einer Umkehrphasensäule RP 18 gereinigt. Das Startmaterial (5,2 mg) und die Hauptfraktionen des gelben Pigmentes wurden gesammelt und getrocknet mit einer Ausbeute von 24,1 mg bzw. 48 % an *Cj*-YCC-2.

Strukturanalyse und spektroskopische Charakterisierung zeigten, dass *Cj*-YCC-2 als weniger polare Verbindung im Vergleich zu *Cj*-NCC-1 im UV/VIS Spektrum in Methanol drei charakteristische Maxima bei 426 nm bei 310 nm und 244 nm (Nanometern), mit den relativen Intensitäten 1,00 zu 0,69 zu 0,50 (siehe Fig. 3a strichlierte Linie). Die molekulare Formel von *Cj*-YCC-2 wurde mittels Massenspektrometrie als C₃₅H₃₈N₄O₈ aus dem Pseudomolekül-lon bei M/Z = 643,2 analysiert. Im Vergleich zum Ausgangsprodukt *Cj*-NCC-1 ergab sich also, dass das Produkt zwei Wasserstoffatome weniger pro Molekül aufweist. Die genaue Konstitution des *Cj*-YCC-2 wurde mittels ¹H-NMR-Spektren sowie homo- und heteronuklearer 2D-Spektren (ROESY, Rotating-Frame Nuclear Overhauser Spectroscopy, COSY, ¹H,¹³C-HSQC, Heteronuclear Single Quantum Correlation und HMBC, Heteronuclear Multiple-Bond Correlation) analysiert. In den ¹H-NMR Spektren von *Cj*-YCC-2 im CD₃OD bei von 25° C konnten die Signale aller 32 Kohlstoff-gebundenen Wasserstoffatome detektiert werden. Besonders sticht das Singulett von CH(5) = O bei niedrigem Feld, das Spinsystem für eine periphere Vinylgruppe bei intermediärem Feld und 5 Singulett von 4 mit Methylgruppen bei hohem Feld und einem in der Nähe von 3,7 ppm heraus. Aus ¹H, ¹³C heteronuklearen NMR-Korrelation (HSQC,g-HMBC und ROESY-Spektren) von *Cj*-YCC-2 konnte die Zuordnung von ¹H und ¹³C Signalen erzielt werden und die Struktur von *Cj*-YCC-2 als Oxidationsprodukt von *Cj*-NCC-1 zugeordnet werden, welches aus der Entfernung von zwei Wasserstoffatomen aus der C1 und C20 Position von *Cj*-NCC-1 erzielt wurde. Für die Nummerierung der einzelnen Atome siehe Fig. 5b. Die Struktur von *Cj*-YCC-2 wurde weiter mittels ¹H-ROESY Spektroskopie analysiert. Diese Spektren ergaben dass die Doppelbindung zwischen C20 und C1 in Z-Konfiguration vorliegt. Daraus ergibt sich folgende Nomenklatur: (13²*S*,15*R*,20*Z*) - 3¹,3²-Didehydro - 4,5,10,15 - (22,24H) - hexahydro - 13² - methoxycarbonyl - 4,5 - seco - 4,5 - dioxo - phytoporphyrinat.

In analoger Weise wurde *Cj*-YCC-1 das polarere der beiden gelben Pigmente analysiert und als Stereoisomer von *Cj*-YCC-2 identifiziert. Das UV/VIS Spektrum einer Lösung von *Cj*-YCC-1 in Methanol zeigte drei charakteristische Absorptionsbanden mit Maxima bei 440, 313 und 247 Nanometern mit relativen Intensitäten von 0,80 zu 1,0 zu 0,77 (siehe Fig. 3a, durchgezogenen Linie). Die molekulare Formel von *Cj*-YCC-1 wurde mittels Massenspektrometrie als C₃₅H₃₈N₄O₈ aus dem Pseudomolekül-Ion bei M/Z = 643,2 analysiert. Daraus ergab sich, dass das gelbe Tetrapyrrol *Cj*-YCC-1 ebenfalls um zwei Wasserstoffatome pro Molekül verringert ist als das Ausgangsmolekül *Cj*-NCC-1. Die genaue Struktur des gelben Kataboliten *Cj*-YCC-1 wurde mittels ¹H-NMR Spektroskopie sowie Homo- und Heteronuklearer 2-D Spektren (ROESY, ¹H,¹³C - HSQC und HMBC) analysiert. Auch in diesem Fall konnten die Signale aller 32 Kohlenstoffgebundenen Wasserstoffatome ermittelt werden. Das Singulett bei niedrigem Feld von CH(5) = O, das Spinsystem für eine periphere Vinylgruppe bei intermediärem Feld und fünf Singuletts von vier Methylgruppen bei hohem Feld und einem nahe 3,7 ppm ragten heraus. Mittels ¹H,¹³C Heteronuklearer NMR Korrelation (HSQC, g-HMBC und ROESY Spektren) von *Cj*-NCC-1 konnte die vollständige Zuordnung der ¹H und ¹³C Signale erreicht werden und die Konstitution von *Cj*-YCC-1 als Oxidationsprodukt von *Cj*-NCC-1 zugeordnet werden, welches aus der Entfernung von zwei Wasserstoffatomen aus der C1 und C20 Position von *Cj-*NCC-1 resultiert. Die Struktur von *Cj*-YCC-1 wurde weiters mittels ¹H-ROESY Spektren charakterisiert und die Konfiguration der neuen Doppelbindung zwischen C20 und C1 als E bestimmt. Auf dieser Basis wurde das gelbe Oxidationsprodukt als Doppelbindungs- (geometrisches) Isomer von *Cj*-YCC-1 identifiziert und der Name (13²*S*,15*R*,20*E*) - 3',3²-Didehydro - 4,5,10,15 - (22,24H) - hexahydro - 13² - methoxycarbonyl - 4,5 - seco - 4,5 - dioxo - phytoporphyrinat zugeordnet.

Stocker et al. verwendeten zur Quantifizierung der antioxidativen Wirkung des Hämabbauproduktes Bilirubin ein einfaches Testsystem. Die essentielle Fettsäure Linolsäure, eine zweifach ungesättigte Fettsäure, ist sehr empfindlich gegenüber einer Oxidation durch freie Radikale. Derartige Oxidationen können in Form von Kettenreaktionen z.B. in Zellmembranen großflächige Schäden herbeiführen. *In vitro* wurde dieser "oxidative Stress" durch den Einsatz einer Azoverbindung (Radikalstarter) simuliert. Derartige Azoverbindungen bilden ständig, abhängig von der Temperatur durch Abspaltung von Stickstoff (N₂) Kohlenstoffradikale, die wiederum mit den ungesättigten Bindungen der Fettsäuren reagieren können. Es bilden sich Fettsäureradikale die ihrerseits wiederum mit Sauerstoff reagieren. Das führt zur Bildung von Fettsäurehydroperoxiden, die wegen ihrer besonderen Absorptionseigenschaften in UV-Bereich (234 nm) spektroskopisch nachweisbar sind. Abhängig von der Konzentration des Radikalbildners steigt also mit der Zeit die Anzahl der Fettsäurehydroperoxidmoleküle an. Setzt man einen antioxidativ wirksamen Stoff zu, können Radikale abgefangen werden, und die Bildung der Hydroperoxide wird verlangsamt.

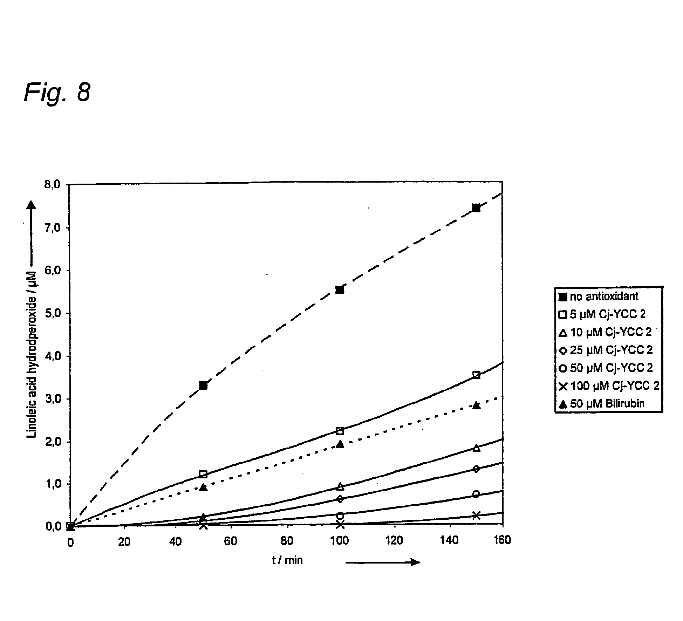

Das Ergebnis dieser Untersuchungen zeigt die Figur 8, in der die zeitliche Abhängigkeit der Zunahme oxidierter Linolsäure bei unterschiedlichen Konzentrationen der Antioxidantien Bilirubin bzw. NCC-1 aufgetragen ist.

Die Aktivität des natürlichen und weniger polaren *Cj*-YCC-2 als Antioxidans wurde in Lösung mittels Standardexperimenten, die unter anderem auch in der WO 2007/030848 beschrieben sind, getestet unter Verwendung der Autoxidation von Linolensäure in der Anwesenheit von Luft und bei Raumtemperatur. Das gelbe Tetrapyrrol *Cj*-YCC-2 inhibierte die Erscheinung von Hydroperoxiden aus einer Radikal-Ketten-Autoxidation von Linolensäure in der Anwesenheit von Luft (siehe Fig. 8). Die Rate der Peroxidbildung wurde bei einer Konzentration c_{0,5} von etwa 10 µM (µMol/Liter) für *Cj*-YCC-1 auf die Hälfte reduziert und es zeigte sich, dass *Cj*-YCC-1 ein deutlich effektiveres Antioxidans als sowohl Bilirubin (C_{0,5} ca. 40 µM) oder *Cj*-NCC-1 (C_{0,5} ca. 100 µM) ist.

Analyse von YCC in seneszenten Blättern von von *Cercidiphyllum japonicum:*
Vier gelbe Blätter von *Cercidiphyllum japonicum* wurden mit flüssigem Stickstoff in einem Mörser gemahlen. 200 µl Methanol und 100 µl Kaliumphosphatpuffer (100 mM, pH 7,0) wurden in einem Reaktionsgefäß den pulverförmigen gelben Blättern hinzugefügt. Das Reaktionsgefäß wurde geschüttelt und anschließend für fünf Minuten bei 13.000 Umdrehungen pro Minute zentrifugiert. Die Lösung wurde abpipettiert und für weitere fünf Minuten zentrifugiert. 20 µl der erhaltenen gelben Lösung wurden auf eine analytische HPLC aufgetragen (HPLC Säule: Thermo, ODS, Hypersil, 250 x 4,5 mm, Partikelgröße 5 µm, Flussrate 500 µl pro Minute, Eluent: t = 0 bis 10 Minuten: 80 % Puffer pH = 7 (100 mM KH₂PO₄/K₂HPO₄), 20 % Methanol; t = 10 bis 70 Minuten: linearer Gradient bis 40 % Puffer und 60 % Methanol; t = 70 bis 94 Minuten: 40 % Puffer und 60 % Methanol.

### Isolierung von NCC und YCC aus frischen seneszenten Blättern von Cercidiphyllum japonicum:

500 g Frischgewicht an gelben von *Cercidiphyllum japonicum* Blättern wurden mit 600 ml Dichlormethan/Methanol 95/5 (v/v) in einem 5 I Stahlbehälter vermischt und mit einem Handmixer (Braun MR 5000) gemixt. Das Lösungsmittel des Breis wurde durch Filtration entfernt. Dies wurde sieben Mal mit 400 ml Portionen von Dichlormethan/Methanol 95/5 (v/v) wiederholt. Die kombinierten Extrakte wurden erneut filtriert und auf eine Silikagel Säule (190 mm Durchmesser, 160 mm Länge, 430 g Silika 60) aufgetragen. Die Säule wurde dann mit 500 ml Dichlormethan gewaschen. Anschließend erfolgte Flüssigchromatographie mittels 500 ml Dichlormethan/Methanol 98/2 (v/v), 500 ml Dichlormethan/Methanol 95/5 (v/v), 250 ml Dichlormethan/Methanol 90/10 (v/v), 1 I Dichlormethan/Methanol 80/20 (v/v), 500 ml Dichlormethan/Methanol 60/40 (vlv) und 500 ml Methanol durchgeführt. Die Chromatographie ergab Rohfraktionen an *Cj*-NCC und *Cj*-YCC, welche kombiniert wurden und mit 0,1 M Kaliumphosphatpuffer (pH 5,2) extrahiert wurden. Die organische Phase wurde abgetrennt und die wässrige Phase zweimal mit 250 ml Dichlormethan extrahiert. Die kombinierten Dichlormethanextrakte wurden filtriert, anschließend wurde das Lösungsmittel verdampft. Der Rückstand wurde in Vakuum getrocknet und ergab 1,897 g Rohprodukt. Das Rohprodukt wurde in 10 ml Methanol gelöst und mit 40 ml 0,1 M Kaliumphosphatpuffer (pH 7,0) verdünnt. Die tieforange Suspension wurde zentrifugiert (4 Minuten bei 4.500 g) und durch ein Glasmikrofaserfilter filtriert (Porengröße 1,6 µm, 25 mm Durchmesser). Das Filtrat wurde auf MPLC aufgetragen (50 ml Injektion, innerer Durchmesser 45 mm, Länge 480 mm, Flussrate 8 ml pro Minute, Gradient: 5 Minuten bei 0,1 M Kaliumphosphatpuffer (pH 7,0) /Methanol 80/20 (v/v), dann innerhalb 20 Minuten auf 0,1 M Kaliumphosphatpuffer (pH 7,0) /Methanol 60/40 (v/v). Die gesammelten NCC und YCC-Fraktionen wurden mittels eines Sep-Pak-Cartridges entsalzt und mit Methanol eluiert. Das Lösungsmittel wurde verdampft und der Rückstand in Vakuum getrocknet um 56,9 mg einer Mischung aus *Cj*-YCC-2 und *Cj-*NCC-2 sowie 478,8 mg *Cj*-NCC-1 zu ergeben.

Die feste Mischung aus *Cj*-YCC-2 und *Cj*-NCC-2 wurde in 10 ml Methanol gelöst und mit 40 ml 0,1 M Kaliumphosphatpuffer (pH 7,0) verdünnt. Die erhaltene stark gelbe Lösung wurde mittels MPLC getrennt (50 ml Injektion, innerer Durchmesser 45 mm, Länge 480 mm, Flussrate 8 ml pro Minute, Gradient: 5 Minuten bei 0,1 Mol/l Kaliumphosphatpuffer (pH 7,0)/Methanol 80/20 (v/v), dann innerhalb 15 Minuten auf 0,1 Mol/l Kaliumphosphatpuffer (pH 7,0)/Methanol 50/50 (v/v). Die gesammelten NCC und YCC-Fraktionen wurden dann mittels eines Sep-Pak-Cartridges entsalzt und die Fraktionen mit Methanol eluiert. Das Lösungsmittel wurde verdampft und die Rückstände in Vakuum getrocknet, um 27,0 mg *Cj*-YCC-2 und 24,9 mg *Cj*-NCC-2 zu ergeben.

Darstellung von *Cj*-YCC-2 mittels Oxidation von *Cj*-NCC-1 auf Silikagel:
Zu einer Lösung von 30,0 mg *Cj*-NCC-1 in 50 ml essigfreiem Dichlormethan wurden 3 g Silikagel zugegeben. Das Lösungsmittel der Aufschlämmung wurde entfernt mittels Filtration und das trockene Pulver in einen Becher transferiert, welcher mit einem Rührer ausgestattet war. Während von außen mittels eines Wasserbades bei Raumtemperatur gekühlt wurde, wurde das gerührte Silikagel Pulver dem Licht einer 100 Watt Wolfram-Glühbirne für 10 Stunden und 15 Minuten ausgesetzt. Das resultierende tieforange Pulver wurde mit 25 ml Methanol extrahiert. Das Lösungsmittel wurde mittels eines Rotationsevaporators bei Raumtemperatur entfernt. Anschließend wurden 1,5 ml Methanol und 1 ml wässriger Phosphatpuffer (0,1 M, pH 7,0) hinzugegeben und die Lösung wurde auf eine präparative HPLC aufgetragen (2 ml Injektionsvolumen, 21,6 mm Säulenlänge, Flussrate 5 ml pro Minute, Gradient: 10 ml bei mM Kaliumphosphatpuffer (pH = 7,0)/Methanol 80:20 (v/v), dann innerhalb von 40 Minuten auf 100 mM Kaliumphosphatpuffer (pH = 7,0)/Methanol 40:60 (v/v). Die gesammelten Fraktionen an *Cj*-YCC-2 wurden mittels einer Sep-Pak-Kartusche entsalzt. Das gelbe Material wurde mit Methanol eluiert und das Lösungsmittel unter Vakuum bei Raumtemperatur verdampft. Das Produkt wurde getrocknet und es ergab eine Ausbeute von 16 mg (53 %) an *Cj*-YCC-2.

Synthese von *Cj*-YCC mittels Oxidation von *Cj*-NCC-1 auf Silikagel:
192.2 mg *Cj*-NCC-1 wurden in 150 ml Dichlormethan gelöst. Eine Suspension von 19,3 g Silikagel in 200 ml Dichlormethan wurden dieser Lösung hinzugefügt. Die Lösung wurde filtriert und der trockene Rückstand in eine Kristallisierschale überführt. Gleichzeitig erfolgt die Kühlung mittels eines Wasserbades. Die Mischung wurde dem Licht einer 100 Watt Wolfram-Glühbirne ausgesetzt (bei einer Distanz von etwa 25 bis 30 cm zum Boden des Bechers). Nach 14 Stunden wurde das gelbe Pulver mit 240 ml Methanol extrahiert. Das Lösungsmittel wurde in Vakuum verdampft und es ergab 179,2 mg an einem orange-rotem Rohprodukt. Dieses Rohprodukt wurde in 20 ml Methanol gelöst und mit 30 ml 0,1 M Kaliumphosphatpuffer (pH 7,0) verdünnt. Die tieforange Lösung wurde filtriert und auf MPLC injiziert (15 ml Injektionsvolumen, innerer Durchmesser 45 mm, Länge 480 mm, Fluss 8 ml pro Minute). Fraktionen an *Cj-*YCC-1, *Cj*-NCC-1 und *Cj*-YCC-2 wurden mit Methanol zu 0,1 M Kaliumphosphatpuffer (pH 7,0; 60/40 v/v) verdünnt. Die gesammelten Fraktionen wurden auf 40 % des Volumens mittels eines Rotationsevaporators eingeengt und mit einem Sep-Pak-Cartridge entsalzt. Anschließend erfolgt die Elution mittels 25 ml Methanol. Das Lösungsmittel wurde verdampft und der Rückstand in Vakuum getrocknet. Auf diese Weise konnten 29 mg *Cj*-YCC-1 (15,1 % Ausbeute), 33,5 mg *Cj*-NCC-1 (17,4 % Rückgewinnung) und 30,3 mg *Cj*-YCC-2 (15,7 % Ausbeute) als gelbes, weißes und gelbes Pulver isoliert werden.

Darstellung von *Cj*-YCC-2 mittels Oxidation von *Cj*-NCC-1 mit DDQ:
50 mg *Cj*-NCC wurden in 5 ml Aceton gelöst (Aceton wurde vorher von Sauerstoff befreit) und die Lösung auf -70° C gekühlt. Anschließend wurden 44,4 µl Essigsäure hinzugegeben. 19,5 mg DDQ in 2,5 ml Aceton gekühlt auf -70°C wurden langsam dem Reaktionsgemisch mittels einer Spritze hinzugefügt. Die resultierende rot gefärbte Reaktionsmischung wurde unter Argonschutzatmosphäre gehalten und in einem Zeitraum von 2 Stunden auf -47°C erwärmt. Die Reaktionsmischung wurde anschließend mit 192 mg Natriumacetat versetzt und für 30 Minuten bei etwa -50°C gehalten. Die Reaktionsmischung wurde dann mit 250 ml 0,1 M Kaliumphosphatpuffer pH 7 versetzt und durch ein Sep-Pak-Cartridge filtriert. Die Reaktionsprodukte wurden mit 50 ml Methanol eluiert und getrocknet. Die rohen Produkte wurden dann mit Methanol gelöst und mit 2 ml Trifluoressigsäure bei Raumtemperatur behandelt. Nach vier Minuten wurde die Mischung mit 250 ml 0,1 M Kaliumphosphatpuffer pH 7,0 durch Filtration über ein Sep-Pak-Cartridge entsalzt. Nach Elution mit 50 ml Methanol wurde das Rohprodukt getrocknet. Die Ausbeute betrug 52,2 mg. Das Reaktionsprodukt wurde mittels MPLC gereinigt auf einer Umkehrphasensäule RP-18. 5,2 mg an *Cj-*NCC-1 konnten rückgewonnen werden. Die Hauptfraktion an *Cj*-YCC-2 wurde gesammelt und getrocknet und ergab 24,1 mg Ausbeute (48,2 % Ausbeute identifiziert mittels HPLC, UV-Vis und ¹H-NMR-Spektrum).

### Experimentelle Details:

Materialien: Die Lösungsmittel für die Extraktionsschritte waren vom Einheitsgrad "reagent-grade" und wurden vor der Benutzung destilliert. Methanol stammte von Merck Darmstadt, Deutschland und Acros Organics, Geel, Belgien und war von HPLC-Qualität. 99 %-iges Bilirubin und 99 %-ige Linolsäure wurden ebenfalls von Acros Organics bezogen, Aluminiumoxid (basisch) für Chromatographie, Chloroform puriss. p.a., Kaliumdihydrogenphosphat puriss. p.a. und Kaliumphosphat dibasisch wasserfrei puriss. p.a. stammten von Fluka, Buchs, Schweiz. Sep-Pak-C18 Cartridges stammten von Waters Associates. Die pH-Messungen wurden mit einer WTW Sentix 21 Elektrode angeschlossen an ein WTW pH535 digital pH Meter durchgeführt. Das pflanzliche Material stammte von Blättern von *Cercidiphyllum japonicum,* gesammelt im Botanischen Garten der Universität Innsbruck im September 2006, gelagert bei -80°C.

HPLC: Gynotek HPLC System mit manueller Probeentnahmevorrichtung, M480 Pumpe (analytisch), M300 Pumpe (präparativ), Phenomenex DG-301 online Entgaser, UVD 340 Diodenanordnungsdetektor und Jasco FP-920 Fluoreszenzdetektor. Die Daten wurden mittels Gynkosoft 5.50 gesammelt und mit Chromeleon V6.50 verarbeitet. HP 1100 System mit manueller Probenentnahme, online Entgaser und Diodenanordnungsdetektor. Die Daten wurden gesammelt und verarbeitet mit HP Chemstation for 3D. LC Packings Ultimate Nano-HPLC-System mit Dionex UVD 340S Diodenanordnungsdetektor für die LC-MS Experimente. Die Daten wurden gesammelt und verarbeitet mit Chromeleon V6.50. Analytische HPLC: Hypersil ODS 5µm 250 x 4,6 mm im Durchmesser Säule bei Raumtemperatur geschützt durch eine Phenomenex ODS 4 mm x 3 mm im Durchmesser Vorsäule, Flussrate 0,5 ml pro Minute, 20 µl Injektionsvolumen. Lösungsmittel A: 100mM Kaliumphosphatpuffer (pH 7,0), Lösungsmittel B: Methanol. Präparative HPLC: Hypersil ODS 5 µm 250 mm x 21,2 mm im Durchmesser Säule bei Raumtemperatur, Flussrate 5 ml pro Minute, die Lösungsmittel wurden durch ein Ultraschallbad entgast. Nano-HPLC: Nucleosil 125-5 50 mm x 100 µm im Durchmesser Kapillarensäule bei Raumtemperatur, Flussrate 300 µl pro Minute, 1 µl Injektionsvolumen. Lösungsmittel A: Wasser, Lösungsmittel B: Methanol.
UV/Vis: Hitachi U-3000 Spektrophotometer; λₘₐₓ(nm)/(rel.ε).
NMR: Varian Unityplus 500 MHz; δ(H) in ppm referenziert auf δ (C¹HD₂OD)=3,31 ppm, Kopplungskonstanten J in Hz, die Spektren wurden bei 26°C aufgezeichnet.
MS: Finigan MAT 95-S in Positiv-Ionen Modus; FAB-MS mit ESI Quelle, positive Ionen, Flussrate 2ml pro Minute, Sprühspannung 3.0 kV, Lösungsmittel Wasser/Methanoi 1:1 (v/v).

Bestimmung der Wirkung von *Cj*-YCC-2 als Antioxidans (nach R. Stocker, Y. Yamamoto, A. F. McDonagh, A. N. Glazer, B.N. Ames, Science 235, 1043 (1987)). Stocklösungen von Linolsäure, Azo-bis-isobutyronitril (AIBN), *Cj*-YCC-2 und Bilirubin wurden hergestellt, indem 470 µl Linolsäure in 1530 µl Chloroform, 9.2 mg AIBN in 1840 µl Chloroform, 0,3 mg Bilirubin in 1500 µl Chloroform 0,8 mg *Cj*-YCC-2 in 700 µl Methanol gelöst wurden. Die genauen Konzentrationen von NCC-1 und Bilirubin in den Stocklösungen wurden mittels UV/Vis Spektroskopie bestimmt (50 µl der Stocklösung wurden mit 3 mL Methanol verdünnt; der Extinktionskoeffizient ε von *Cj*-YCC-2 in Methanol bei 426 nm beträgt 32600 (ε von Bilirubin in Methanol bei 450 nm beträgt 55000, siehe Referenz 18). Die Reaktionslösungen wurden hergestellt, indem Aliquote der Stocklösungen zu den folgenden Konzentrationen gemischt wurden: 0,15 M Linolsäure, 2 mM AIBN und 0 - 100 µM *Cj*-YCC-2 bzw. Bilirubin. Die Mischungen enthielten 6 % (v/v) Methanol. Die Reaktionslösungen wurden bei 37°C gehalten, die Bildung von Linolsäure Hydroperoxid wurde mittels UV/Vis Spektroskopie bzw. HPLC Analyse bestimmt.

UV/Vis Spektroskopie: 40 µl der Reaktionsmischung wurden mit 3 mL Methanol verdünnt. Die Absorption wurde gegen Methanol gemessen, die Gesamtabsorption bei 234 nm wurde zur Bestimmung herangezogen (siehe Fig. 8).

Analytische HPLC wurde auf einer 250 mm x 4,6 mm im Durchmesser Hypersil ODS 5 µm Säule bei Raumtemperatur durchgeführt.
Das Eluierungsmittel für die isokratische Trennung war Methanol mit einer Flussrate von 1 mL pro Minute. 40 µl der Reaktionsmischung wurden mit 1,5 mL Chloroform verdünnt, 20 µl dieser Lösung wurden auf die HPLC aufgetragen.

### Spektroskopische Daten:

*Cj*-YCC-2 **(2)**. UV/Vis (λₘₐₓ, log ε, Methanol, c = 1.59*10⁻⁵ M): 426 (4.51), 310 (4.35), 244 (4.22) (Fig. 3). ¹H-NMR (500 MHz, in CD₃OD): δ = 2.20 (s, H₃C(2¹)), 2.13 (s, H₃C(12¹)), 2.15 (s, H₃C(18¹)), 2.23 (s, H₃C(7¹)), 2.35 (m, H₂C(17²)), 2.63 (m, H₂C(8¹)), 2.71 (m, H_{A}C(17¹)), 2,79 (m, H_{B}C(17¹)), 3.48 (m, H₂C(8²)), 3.77 (s, H₃C(13⁵)), 3.96 (s, H₂C(10)), 5.05 (s, HC(15)), 5.35(dd, J = 2/11.5 Hz, H_{A}C(3²)), 6.11 (d broad, J = 17.5 Hz, H_{B}C(3²)), 6.21 (s, HC(20)), 6.55 (m, HC(3¹)). ¹³C-NMR (in CD₃OD, Signalzuordnung mittels ¹H¹³C-HSQC und ¹H¹³C-HMBC Experimente): δ = 9.01 (7¹), 9.28 (12¹), 9.49 (2¹), 9.52 (18¹), 22.0 (17¹), 23.6 (10), 28.0 (8¹), 37.1 (15), 40.0 (17²), 52.7 (13⁵), 62.6 (8²), 67.3 (13²), 102.6 (18), 102.6 (20), 112.2 (12), 118.1 (3²), 120.7 (8), 124.1 (17), 125.0 (3),125.5 (19),125.8 (13), 129.3 (3¹), 129.6 (6), 131.4 (2), 131.6 (16), 134.9 (11), 138.4 (9), 143.1 (1), 159.9 (14), 171.1 (13³), 182.0 (17³). ESI-MS: m/z = 1323.43 (18, [2M+K+H]⁺), 1307.58 (20, [2M+Na+H]⁺), 683.20 (10), 682.20 (16), 681.21 (30, [M+K]⁺), 680.16 (9), 645.20 (17), 644.20 (39), 643.21 (100, [M+H]⁺), 642.17 (38), 641.18 (16), 613.17 (7), 612.18 (21), 611.19 (43), 610.20 (27), 609.17 (14), 520.11 (14, [M+H-Ring A]⁺), 490.12 (13, [M+H-Ring B]⁺), 458.09 (25), 357.07 (25), 325.15 (50).
*Cj*-YCC-1 (1). UV/Vis (λₘₐₓ, rel. ε, Methanol): 440 (0.80), 313 (1.00), 247 (0.77) (Fig. 3). ¹H-NMR (500 MHz, in CD₃OD): δ = 1.67 (s, H₃C(2¹)), 2.03 (s, H₃C(18¹)), 2.15 (s, H₃C(12¹)), 2.26 (s, H₃C(7¹)), 2.37 (m, H₂C(17²)), 2.68 (m, H₂C(8¹)), überlagert bei 2.70 (m, H_{A}C(17¹)), 2,80 (m, H_{B}C(17¹)), 3.51 (m, H₂C(8²)), 3.76 (s, H₃C(13⁵)), 3.95 (s, H₂C(10)), 4.98 (s, HC(15)), 5.38(dd, J = 2.5/11.5 Hz, H_{A}C(3²)), 6.15 (dd, J = 2.5/18 Hz, H_{B}C(3²)), 6.37 (s, HC(20)), 6.51 (dd, J = 12/17.5 Hz, HC(3¹)), 9.42 (s, HC(5)). ¹³C-NMR (in CD₃OD, Signalzuordnung mittels ¹H¹³C-HSQC und ¹H¹³C-HMBC Experimente): δ = 9.29 (12¹), 9.39 (7¹), 9.59 (18¹), 12.2 (2¹), 22.1 (17¹), 23.5 (10), 27.8 (8¹), 37.0 (15), 40.1 (17²), 52.1 (13⁵), 62.6 (8²), 107.9 (20), 112.5 (12), 119.3 (3²), 121.1 (8), 123.2 (17), 124.0 (13), 124.7 (19), 129.4 (16), 129.6 (3), 129.6 (6), 134.6 (11), 137.1 (1), 138.4 (9), 140.5 (2), 161.4 (14), 171.6 (13³), 182.3 (17³). ESI-MS: m/z = 1323.49 (10, [2M+K+H]⁺), 1307.58 (5, [2M+Na+H]⁺), 719.04 (20, [M-H+2K]⁺), 682.12 (49), 681.13 (100, [M+K]⁺), 666.13 (10), 665. 1 (35, [M+Na]⁺), 644.13 (5), 643.20 (15, [M+H]⁺).

Ergänzende Literatur:
(1) S. B. Brown, J. D. Houghton. G. A, F. Hendry, in Chlorophylls (Ed.: H. Scheer), CRP-Press; Boca Raton. USA, 1991, pp. 405-439.
(2) P. Matile, Chimia 1987, 41, 376-381.
(3) B. Kräutler, B. Jaun, K. Bortlik. M. Schellenberg, P. Matile, Angew. Chcm. Int. Ed. 1991, 30, 1315-1318.
(4) P. Matile, S. Hörtensteiner, H. Thomas, B. Kräutler, Plant Physiol. 1996, 112, 1403-1409.
(5) B. Kräutler, S. Hörtensteiner, in Chlorophylls and Bacteriochlorophylls: Biochemistry, Biophysics, Funktions and Applications Vol 25 (Eds.: B. Grimm, R. Porra, W. Rüdiger, H. Scheer). Springer, Dordrecht, The Netherlands 2006, pp. 237-260.
(6) P. Matile, in Regulation of Photosynthesis (Eds.: E.-M. Aro, B. Andersson), Kluwer Academic Publishers, Dordrecht, The Netherlands, 2001, pp. 277-296.
(7) B. Kräutler; P. Matile, Accounts Chem Res 1999, 32, 35-43.
(8) P. Matile, in Advances in Botanical Research, Vol. 25 (Ed.: J. A. Callow), Academic press, New York, 1997, pp. 87-1 12.
(9) T. Müller, M. Ulrich, K. H. Ongania, B. Kräutler, Angew. Chern. Int. Ed. 2007, 46, 8699-8702.
(10) K. Bortlik, C. Peisker, P. Matile, J. Plant Physiol. 1990, 136, 161-165.
(11) R. Stocker, Y. Yamatnoto, A. F. Mcdonagh, A. N. Glazer, B. N. Ames, Science 1987,235, 1043-1 046.
(12) C. Curty, N. Engel, Phytochemistry 1996, 42, 153 1-1536.
(13) M. Oberhuber, J. Berghold, K. Breuker, S. Hörtensteiner, B. Kräutler, Proc. Natl. Acad. Sci. USA 2003, 100, 6910-6915.
(14) F. G. Losey, N. Engel, J. Biol. Chem. 2001, 276, 8643-8647.
(15) P. R. Ortiz de Montellano, K. Auclair, in The Iron and Cobalt Pigments: Biosynthesis, Structure, und Degradation, Vol. 12 (Eds.: K. M. Kadish, K. M Smith, R. Guilard), Academic Press, Amsterdam, 2003, pp. 183-210.
(16) N. Frankenberg, J. C. Lagarias, in The Porphyrin Handbook, Vol. 13 (Eds.:K. M. Kadish, K. M. Smith, R. Guilard), Elsevier Science; Oxford, UK, 2003, pp. 21 1-235.
(17) D. E. Baranano. M. Rao, C. D. Ferris, S. H. Snyder, Proc. Natl. Acad. Sci. USA 2002, 99: 16093-16098.
(18) G. Agati, F. Fusi, J. Photochem. Photobiol. B 1990, 7, 1-14.
(19) H. Scheer, in Chlorophylls (Ed.: H. Scheer), CRC Press, Boca Raton. USA, 1991, pp. 3-30.

## Patentansprüche

1. Verbindung der Formel (I) 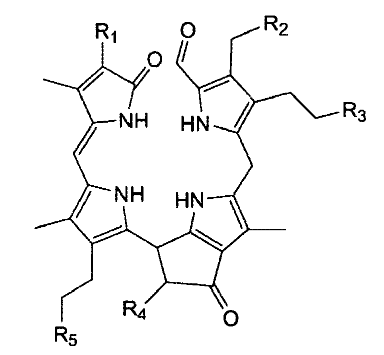 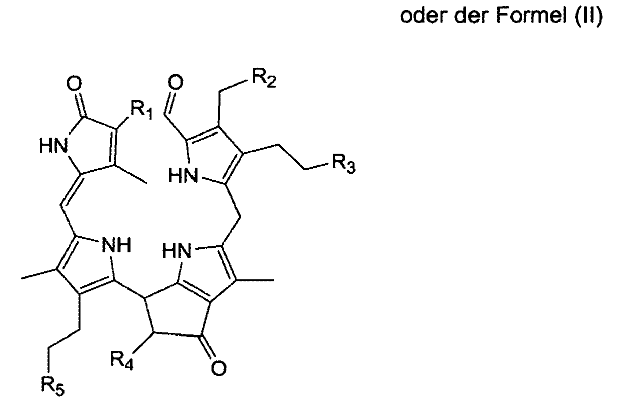 oder Stoffgemisch daraus, oder ein pharmakologisch, dermatologisch oder kosmetisch verträgliches Salz oder Solvat davon, wobei R₁ bis R₅ ausgewählt ist aus den Resten
R₁: -Alkyl, -Vinyl, -CHOH-CH₂OH, -CH(OAcyl)-CH₂OH, -CHOH-CH₂(OAcyl), -CH(OAcyl)-CH₂(OAcyl), 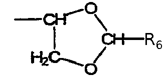 mit R₆ = -Alkvl oder -Aryl,
R₂: -H, -OH, -OAlkyl, -OAcyl, -Saccharidreste, -acylierte Saccharidreste
R₃: -H, -OH, -OAlkyl, -OAcyl,-OAcyl acyliert, Saccharidreste, acylierte Saccharidreste
R₄, R₅: -COOH, -Carbonsäureester

2. Verbindung nach Anspruch 1, **dadurch gekennzeichnet, dass** R₁ gleich Vinyl (-CH=CH₂) oder -CHOH-CH₂OH, R₂ gleich -H oder -OH, R₃ gleich -H oder -OH oder ein Saccharidrest , R₄ gleich COOH oder COOCH₃ und R₅ gleich -COOH ist.

3. Verbindung nach Anspruch 1 oder Anspruch 2 zur Verwendung als Arzneimittel.

4. Verbindung nach Anspruch 1 oder Anspruch 2 zur Verwendung als Arzneimittel zur Vorbeugung oder Behandlung von Krebserkrankungen.

5. Verbindung nach Anspruch 4, **dadurch gekennzeichnet, dass** die Krebserkrankung aus der Gruppe Hautkrebs, Brustkrebs, Lungenkrebs, Darmkrebs oder Leukämie stammt.

6. Verbindung nach Anspruch 1 oder Anspruch 2 als Antioxidationsmittel.

7. Verbindung nach Anspruch 1 oder Anspruch 2 als Konservierungsmittel.

8. Verbindung nach Anspruch 1 oder Anspruch 2 als Nahrungsergänzungsmittel.

9. Verbindung nach Anspruch 1 oder Anspruch 2 als kosmetisches Mittel.

10. Verwendung einer Verbindung nach Anspruch 1 oder Anspruch 2 zur Herstellung eines Arzneimittels.

11. Verwendung nach Anspruch 10, **dadurch gekennzeichnet, dass** das Arzneimittel zur Vorbeugung und/oder Behandlung von Krebserkrankungen verwendet wird.

12. Verfahren zur Herstellung einer Verbindung nach Anspruch 1 oder Anspruch 2 sowie dessen Salze und Solvate, umfassend die Schritte:
Oxidation einer Verbindung mit der Formel (III) 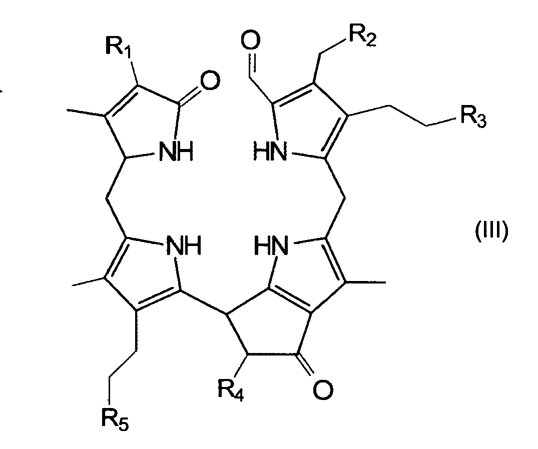 wobei R₁, R₂, R₃, R₄ und R₅ dieselben Reste sind wie in der Verbindung der Formel (I) oder (II).

13. Verfahren nach Anspruch 12, **dadurch gekennzeichnet, dass** die Verbindung der Formel (III) in Anwesenheit von Sauerstoff oxidiert wird.

14. Verfahren nach Anspruch 12 oder Anspruch 13, **dadurch gekennzeichnet, dass** die Oxidation in Anwesenheit von Licht durchgeführt wird.

15. Verfahren nach einem der Ansprüche 12 bis 14, **dadurch gekennzeichnet, dass** als Oxidationsmittel ein Benzochinon, vorzugsweise 2,3-Dichlor-5,6-dicyano-p-benzochinon, eingesetzt wird.

16. Verfahren zur Gewinnung einer Verbindung nach Anspruch 1 oder Anspruch 2 sowie dessen Salze und Solvate, aus pflanzlichem Material, insbesondere aus Pflanzenblättern, **gekennzeichnet durch** die Schritte:
• Extraktion des pflanzlichen Materials mit einem Lösungsmittel zu einem Rohextrakt
• Chromatographie dieses Extraktes
• Abziehen des Lösungsmittels und gegebenenfalls Kristallisation des erhaltenen Rückstandes.

## Claims

1. A compound of the formula (I) 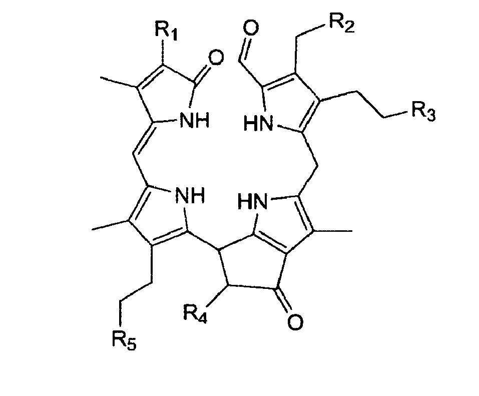 or of the formula (II) 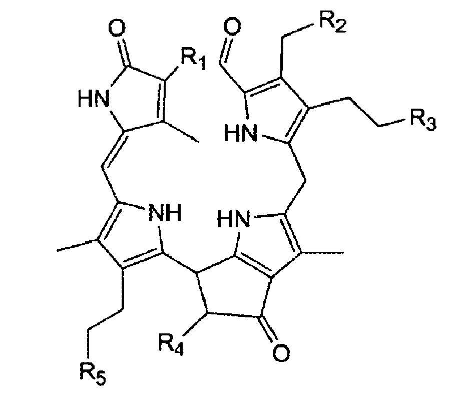 or a mixture of substances thereof, or a pharmacologically, dermatologically or cosmetically acceptable salt or solvate thereof, wherein R₁ to R₅ are selected from the radicals
R₁: -alkyl, -vinyl, -CHOH-CH₂OH, -CH(O-acyl)-CH₂OH, -CHOH-CH₂(O-acyl), -CH(O-acyl)-CH₂(O-acyl), 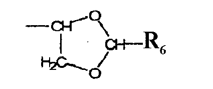 with R₆ = -alkyl or -aryl,
R₂: -H, -OH, -O-alkyl, -O-acyl, -saccharide radicals, acylated saccharide radicals
R₃: -H, -OH, -O-alkyl, -O-acyl, -O-acyl acylated, saccharide radicals, acylated saccharide radicals
R₄, R₅: -COOH, -carboxylic acid ester

2. A compound according to claim 1, **characterized in that** R₁ is vinyl (-CH=CH₂) or -CHOH-CH₂OH, R₂ is -H or -OH, R₃ is -H or -OH or a saccharide radical, R₄ is COOH or COOCH₃ and R₅ is -COOH.

3. A compound according to claim 1 or claim 2 for the use as a drug.

4. A compound according to claim 1 or claim 2 for the use as a drug for the prevention or treatment of cancers.

5. A compound according to claim 4, **characterized in that** the cancer pertains to the group consisting of skin cancer, breast cancer, lung cancer, colon cancer or leukemia.

6. A compound according to claim 1 or claim 2 as an antioxidant.

7. A compound according to claim 1 or claim 2 as a preservative.

8. A compound according to claim 1 or claim 2 as a nutritional supplement.

9. A compound according to claim 1 or claim 2 as a cosmetic product.

10. The use of a compound according to claim 1 or claim 2 for the preparation of a drug.

11. The use according to claim 10, **characterized in that** the drug is used for the prevention and/or treatment of cancers.

12. A method for the preparation of a compound according to claim 1 or claims 2 as well as of salts and solvates thereof, comprising the following steps:
oxidation of a compound of the formula (III) 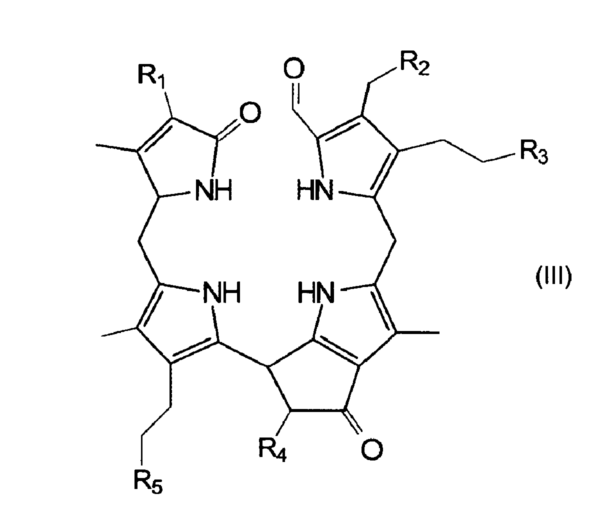 wherein R₁, R₂, R₃, R₄ and R₅ are the same radicals as in the compound of the formula (I) or (II).

13. A method according to claim 12, **characterized in that** the compound of the formula (III) is oxidized in the presence of oxygen.

14. A method according to claim 12 or claim 13, **characterized in that** the oxidation is carried out in the presence of light.

15. A method according to any of claims 12 to 14, **characterized in that** there is used as oxidant a benzoquinone, preferably 2,3-dichloro-5,6-dicyano-p-benzoquinone.

16. A method for the preparation of a compound according to claim 1 or claim 2 as well as salts and solvates thereof from plant material, in particular from plant leaves, **characterized by** the following steps:
• extraction of the plant material with a solvent into a crude extract
• chromatography of said extract
• withdrawal of said solvent and optionally crystallization of the residue obtained

## Revendications

1. Composé de formule (I) 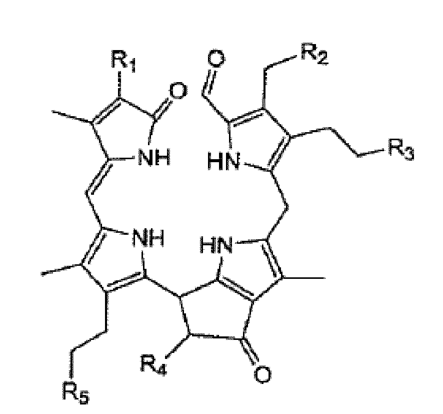 ou de formule (II) 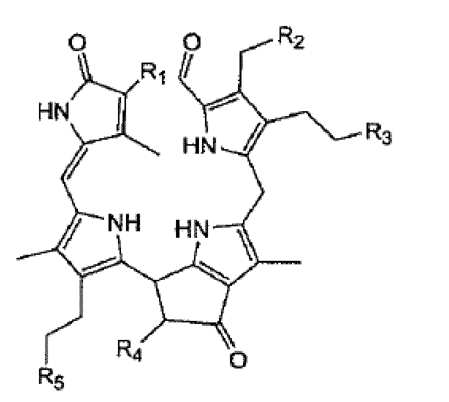 ou mélanges de ces substances, sel pharmacologique, dermatologique ou cosmétiquement compatible ou solvat de ces composés, dans lesquels R₁ à R₅ sont sélectionnés parmi les groupes :
R₁ : -alkyle, -vinyle, -CHOH-CH₂OH, -CH(O-Acyl)-CH₂OH, -CHOH-CH₂(O-Acyl), -CH(O-Acyl)-CH₂(O-Acyl), 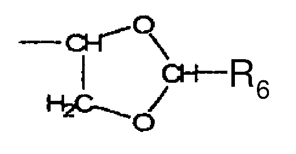 avec R₆ = -alkyle ou -aryle
R₂ : -H, -OH, -O-alkyle, -O-acyle, groupes saccharide ou saccharide acylé,
R₃ : -H, -OH, -O-alkyle, -O-acyle, -O-acyle acylés, groupes saccharide, groupes saccharide acylés, R₄, R₅ : -COOH, -ester d'acide carboxylique.

2. Composé selon la revendication 1, **caractérisé en ce que** R₁ représente un vinyle (-CH=CH₂) ou -CHOH-CH₂OH, R₂ représente -H ou -OH, R₃ représente -H ou -OH ou un groupe saccharide, R₄ représente COOH ou COOCH₃ et R₅ représente -COOH.

3. Composé selon la revendication 1 ou la revendication 2, destiné à être utilisé comme médicament.

4. Composé selon la revendication 1 ou la revendication 2, destiné à être utilisé pour prévenir ou traiter des maladies cancéreuses.

5. Composé selon la revendication 4, caractérisé en de que la maladie cancéreuse provient de l'ensemble des cancers de la peau, du sein, du poumon, de l'intestin ou de la leucémie.

6. Composé selon la revendication 1 ou la revendication 2, destiné à être utilisé comme agent antioxydant.

7. Composé selon la revendication 1 ou la revendication 2, destiné à être utilisé comme agent de conservation.

8. Composé selon la revendication 1 ou la revendication 2, destiné à être utilisé comme complément alimentaire.

9. Composé selon la revendication 1 ou la revendication 2, destiné à être utilisé comme agent cosmétique.

10. Utilisation d'un composé selon la revendication 1 ou la revendication 2 pour la préparation d'un médicament.

11. Utilisation selon la revendication 10, **caractérisé en ce que** le médicament est utilisé pour prévenir et/ou traiter des maladies cancéreuses.

12. Procédé de fabrication d'un composé selon la revendication 1 ou la revendication 2 ainsi que de ses sels et solvats, le procédé comprenant les étapes qui consistent à :
oxyder un composé de formule (III) 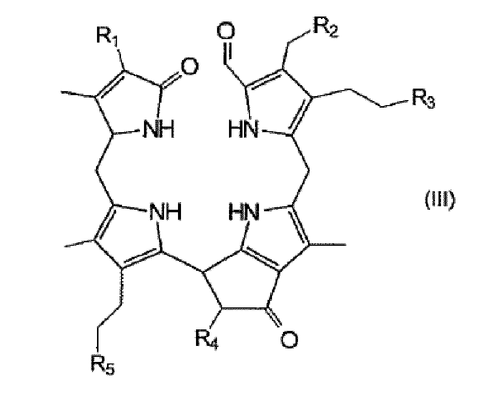 dans laquelle R₁, R₂, R₃, R₄ et R₅ représentent les mêmes groupes que dans le composé de formule (I) ou de formule (II).

13. Procédé selon la revendication 12, **caractérisé en ce que** le composé de formule (III) est oxydé en présence d'oxygène.

14. Procédé selon la revendication 12 ou la revendication 13, **caractérisé en ce que** l'oxydation est réalisée en présence de lumière.

15. Procédé selon l'une des revendications 12 à 14, **caractérisé en ce qu'**il utilise comme agent oxydant une benzoquinone et de préférence la 2,3-dichloro-5,6-dicyano-p-benzoquinone.

16. Procédé de tension d'un composé selon la revendication 1 ou la revendication 2 ainsi que de ses sels et solvats à partir de matériau végétal, en particulier de feuilles de plantes, le procédé étant **caractérisé par** les étapes qui consistent à :
- extraire du matériau végétal un extrait brut à l'aide d'un solvant,
- chromatographier cet extrait,
- séparer le solvant et éventuellement cristalliser le résidu ainsi obtenu.
